# EUROPEAN PATENT SPECIFICATION

(11) **EP 3 889 603 B1**
(45) Date of publication and mention of the grant of the patent: **22.01.2025**
(21) Application number: 18941475.8
(22) Date of filing: 01.12.2018
(51) Int. Cl.: G01N 33/53, G01N 33/50, G01N 1/30

(54) **PREPARATION METHOD FOR LYMPHOCYTE SAMPLE FOR FLOW CYTOMETRY ANALYSIS**
VERFAHREN ZUR HERSTELLUNG EINER LYMPHOZYTENPROBE FÜR EINE DURCHFLUSSZYTOMETRISCHE ANALYSE
PROCÉDÉ DE PRÉPARATION D'UN ÉCHANTILLON DE LYMPHOCYTE POUR ANALYSE PAR CYTOMÉTRIE DE FLUX

(43) Date of publication of application: 06.10.2021
(73) Proprietor: Mingdao Innovation (Beijing) Medical-Tech Co., Ltd., Beijing 101111 (CN)
(72) Inventor: YAO, Weili, Beijing 101111 (CN)
(74) Representative: Michalski Hüttermann & Partner Patentanwälte mbB
(86) International application number: PCT/CN2018/118801
(87) International publication number: WO 2020/107495

(56) References cited:
- CN-A- 106 771 242
- CN-A- 107 063 982
- CN-A- 108 508 196
- XIANG ZHOU ET AL: "Establishment and Evaluation of Flow Cytometry Method for Isolation of T Lymphocytes from Peripheral Blood Mononuclear Cells", PROGRESS IN MODERN BIOMEDICINE, vol. 17, no. 21, 31 July 2017 (2017-07-31), pages 4016 - 4018, XP009521353, DOI: 10.13241/j.cnki.pmb.2017.21.004

## Description

### Technical Field

The present invention relates to the field of medicines and particularly provides a method for preparing lymphocyte samples, specifically a method for preparing lymphocyte samples from immune cells for flow cytometry analysis.

### Background

CN 108508196 A refers to a kit for detecting human regulatory T cell subtypes and a detection method and belongs to the technical field of cell subtype detection. The provided kit comprises components as follows: a blood diluent, a mononuclear cell separation medium, a cell culture fluid, a lymphocyte activation solution, dead cell removal dyes, an FcR blocking agent, a fluorescence labeled antibodyresisting human cell surface labeling molecules, a fluorescence labeled antibody resisting human intracellular molecules, a PBS buffer solution, lipopolysaccharide, a washing buffer solution, a celldyeing buffer solution, a cell fixing solution and a permeable membrane lotion. During detection, firstly, mononuclear cells in whole blood are separated, then, mononuclear cells of human peripheral blood are stimulated, finally, a fluorescent antibody is stained, and the regulatory T cell subtypes can be detected.

CN 107063982 A refers to a method for detecting avian peripheral blood T lymphocyte subsets by flow cytometry; the method comprises the steps of collecting avian peripheral venous blood, preparing anticoagulant blood, and treating with lymphocyte separate to obtain peripheral blood leukocytes; centrifugally washing the peripheral blood leukocytes with PBS phosphate buffer solution to prepare single-cell suspension; sucking the single-cell suspension, adding anti-chicken CD3, CD4 and CD8 monoclonal antibodies, mixing well by cyclone, and staining in the shade at 4 °C; after staining, washing with PBS, resuspending cells, and detecting with a flow cytometer; analyzing detection results, the ratio of avian peripheral blood T lymphocyte subsets.

A flow cytometric sample is mainly used in blood sample detection, while the preparation of a cell sample for use in a conventional flow cytometry detection usually adopts the following methods: (1) analyzing the influence of different treatment methods of peripheral blood on the results of a flow cytometric analysis; and (2) detecting T-lymphocyte subsets phenotype in 25 lung cancer patients by using flow cytometry, including adding corresponding volume of antibody to a flow tube, then adding 50 or 100 µl of anticoagulant peripheral blood samples, mixing, incubating without light at room temperature for 20 minutes, adding 1×hemolysin (i.e. red blood cell lysis solution), respectively, and mixing before detection with a flow cytometer. The current method for preparing flow cytometric sample mainly includes incubating the peripheral blood with antibody for 20-30 minutes, adding red blood cell lysis solution, performing lysis for 5-15 minutes, and then directly performing detection on a flow cytometer, or detecting on a flow cytometer after washing once. The preparation method suffers from the following problems. The first problem is the incubation time after adding the red blood cell lysis solution. Sufficient amount of hemolysin is needed based on the number of red cells, normally by observing whether the solution is clear after adding hemolysin. If it is clear, it means that the red blood cell is completely lysed, but it is easy to cause damage to lymphocyte. If the time of the lysis is insufficient, the red blood cell cannot be completely lysed, which will influence the results of detection and analysis. The second problem lies in that the number of lymphocyte samples cannot be strictly controlled. If the same volume of antibody is added in spite of the number of lymphocytes in the original blood sample, it is easy to cause oversaturated or insufficient amount of antibody. If the number of cell samples to be detected is small, trace amount of cell subtype is difficult to be detected or inaccurately detected due to small number of samples, which cannot accurately reflect the actual situation of some lymphocyte subgroups. When the number of cell samples is too large, for example when the lymphocytes of some patients are above a standard level, the amount of the antibody will be insufficient, which will lead to a lower detection result. The third problem lies in that, the sample obtained from the lysis has too much debris, and the background is not clean, which will affect the collection and analysis of flow cytometer data, and may cause bias to the adjustment of lymphocyte compensation.

CN 201410486089.7 discloses a method and kit for lymphocyte immunotyping. The method includes adding mixed antibody into two flow tubes, adding 50µl anticoagulant peripheral blood samples, fully mixing, incubating without light at room temperature for 30 minutes, lysing red cells with red cell lysis solution for 5 minutes, adding 1ml PBS to wash once (3500rpm, 2min), adding 200µl PBS, and performing detection on a flow cytometer. This invention also suffers from the above technical problem.

Therefore, it is necessary to develop a new method for preparing lymphocyte samples for use in flow cytometry analysis.

### INVENTION

In view of the status of the current technology, the present invention provides a method for preparing flow cytometric sample in human immune cells in vitro for flow cytometry, which includes the steps of:
1) separating plasma and blood precipitation by centrifuging in vitro anticoagulant blood samples to obtain blood cell precipitate;
2) mixing the blood cell precipitate with PBS solution to provide a blood cell dilution;
3) adding lymphocyte separation solution with qual volume to the final cell solution obtained in step 2) in a centrifuge tube, adding the blood cell dilution into the centrifuge tube containing the lymphocyte separation solution. After centrigfugation, removing plasma and recovering buffy coat cells;
4) adding the buffy coat cells to a centrifuge tube, adding PBS solution under stirring, centrifuging, discarding supernatant, and then adding PBS solution to adjust the cell concentration to provide lymphocyte suspension with concentration of 1×10⁵/ml - 1×10⁸/ml, preferably 1.5×10⁶/ml - 3×10⁶/ml, and most preferably 1.5-2.0×10⁶/ml;
5) adding the lymphocyte suspension into a flow tube respectively, then adding flow cytometric antibody to label lymphocyte subpopulations, mixing evenly, and incubating at 2-8°C without light;
6) adding PBS solution to the flow tube, mixing, and centrifuging to remove unbound antibody;
7) completion of samples
   ① If a detection on a flow cytometer is performed immediately, the supernatant is discarded and the precipitate is mixed with PBS-EDTA solution at 2-8°C for flow cytometric detection;
   ② If the detection cannot be performed immediately, 0.05-5% formalin, preferably 1% formalin, is added, mixed, and left to stand at 2-8°Cwithout light. Before detection, each of the tubes is added with PBS solution, centrifuged to discard the supernatant, and evenly mixed with PBS-EDTA solution at 2-8°C for flow cytometric detection.

As an example, the concentration can be 1.5×10⁶/ml, 1.6×10⁶/ml, 1.7×10⁶/ml, 1.8 ×10⁶/ml, 1.9×10⁶/ml, or 2.0×10⁶/ml.

In the method of the present application, as one of the embodiments, the PBS solution is 0.005-0.05M PBS solution at pH 7.2-7.4. In the method of the present application, as an example, the 0.005-0.05M PBS solution can be 0.005 M PBS, 0.01 M PBS, 0.02 M PBS, 0.03 M PBS, 0.04 M PBS or 0.05 M PBS solution.

As one of the embodiments, alternatively, the PBS buffer solution may contain calf serum and/or EDTA, and the concentration of calf serum or EDTA can be a conventional concentration in the art.

In the method of the present application, as one of the embodiments, the PBS-EDTA solution is a mixed solution at pH 7.2-7.4 containing 0.005-0.05M PBS and a final concentration of 2-3mM EDTA.

In the method of the present application, as one of the embodiments, step 1 of the present application further includes centrifuging conditions of 1500-3500rpm, 5-30min; preferably 1500-2900rpm and 15-20min.

In the method of the present application, as one of the embodiments, in step 2), the blood cell precipitate and PBS solution are added in a volume ratio of 1:0.5-1:2 and preferably 1:1.

In the method of the present application, as one of the embodiments, step 3) of the present application further includes centrifuging conditions of 1500-3500rpm, 10-20min, 4°C; preferably 1500-2900rpm, 20min, 4°C.

In the method of the present application, as one of the embodiments, step 4) of the present application further includes centrifuging conditions of 1500-3500rpm for 5-20 minutes; preferably 1500-2900rpm for 5-10 minutes.

In the method of the present application, as one of the embodiments, step 6) of the present application further includes centrifuging conditions of 1500-2900rpm for 5-10 minutes.

In the method of the present application, as one of the embodiments, the amount of PBS solution added in step 6) is an amount equal to the volume of blood cell precipitate.

In the method of the present application, as one of the embodiments, the PBS solution in step 7) is 0.005-0.05M PBS solution at pH 7.2-7.4; as one of the embodiments, alternatively, the PBS buffer solution can contain calf serum and/or EDTA, and the concentration of the calf serum or EDTA can be a conventional concentration in the art; as one of the embodiments, the PBS-EDTA solution is a mixed solution at pH 7.2-7.4 containing 0.005-0.05M PBS and a final concentration of 2-3mM EDTA.

In the method of the present application, as one of the embodiments, the lymphocyte includes, but not limited to, lymphocyte subpopulations, which can include memory lymphocyte and other lymphocyte subpopulations that need to be detected, as well as functional analysis, cytokine analysis and dendritic cell maturity detection for lymphocyte subpopulations.

As one of the embodiments, the method of the present application includes
(I) separation of human peripheral blood mononuclear cells from peripheral blood
   (1) obtaining an *in vitro* anticoagulant peripheral blood sample;
   (2) centrifuging the blood sample in a centrifuge tube and discarding the upper plasma to provide lower blood cell precipitate;
   (3) diluting the blood cell precipitate with PBS solution by a volume ratio of 1:1 to obtain blood cell diluent;
   (4) adding lymphocyte separation solution having a volume equal to the volume of the blood cell dilution to a centrifuge tube;
   (5) adding the blood cell dilution into the centrifuge tube in step (4) slowly for keeping the layer of the separation solution clear;
   (6) Slowly placing the centrifuge tube into a low-speed centrifuge, balancing, and centrifuging;
   (7) discarding the supernatant after centrifuging to provide buffy coat cells;
   (8) placing the buffy coat cells into a centrifuge tube, adding PBS solution, counting, and centrifuging; and
   (9) removing the supernatant after centrifuging, and adding PBS solution at 2-8°C according to the result of counting, so that the cell density is 1.5-3.0×10⁶/ml, providing a cell suspension.
      As an example, the concentration can be 5×10⁶/ml, 2.0×10⁶/ml, 2.5×10⁶/ml, 3.0× 10⁶/ml.
(II)Preparation of flow cytometric samples
   (1) sample staining
      A flow tube is added with 100 µl lymphocyte suspension, added with flow cytometric antibody for labeling lymphocyte subpopulations, mixed evenly, and incubated at 2-8°C without light in the dark for 10-30 minutes, preferably 15-20 minutes, and most preferably 20 minutes.
   (2) sample washing and solution adding
      After incubation, the tube is added with pre-cooled PBS solution to resuspend the cells, centrifuged at 1500-2900 rpm for 5-10 minutes, mixed evenly, and removed of unbound antibody.

As one of the embodiments, the method of the present application includes
(I) separation of human peripheral blood mononuclear cells from peripheral blood
   (1) obtaining an *in vitro* anticoagulant peripheral blood sample, in which 0.2 ml is counted, and 1 ml is used as a sample, labeled, and stored in a refrigerator at 4°C;
   (2) centrifuging the rest of the blood in a centrifuge tube at 1500-2900 rpm for 20 minutes, and discarding the upper plasma;
   (3) diluting the cell precipitate of the rest of the blood with PBS solution by a volume ratio of 1:1;
   (4) adding lymphocyte separation solution having a volume equal to the volume of the blood cell dilution into a centrifuge tube;
   (5) adding the blood cell dilution into the centrifuge tube in step (4) slowly for keeping the layer of the separation solution clear;
   (6) slowly placing the centrifuge tube into a low-speed centrifuge, balancing, and centrifuging at 1500-2900 rpm for 20 mins;
   (7) discarding upper supernatant slowly by using a pipette after centrifuging;
   (8) pipetting middle buffy coat cells into a new centrifuge tube, adding PBS solution to a volume of 10 ml, counting 200µl of cell suspension on a blood cell counter, and centrifuging the rest of the cell suspension at 1500-2900 rpm for 5-10 mins; and
   (9) discarding the supernatant, absorbing to remove residual liquid after centrifuging, and adding appropriate amount of pre-cooled PBS solution at 2-8°C according to the result of counting, to adjust the cell density to 1.5-2.0×10⁶/ml.

As an example, the concentration can be 1.5×10⁶/ml, 1.6×10⁶/ml, 1.7×10⁶/ml, 1.8 ×10⁶/ml, 1.9×10⁶/ml or 2.0×10⁶/ml.

### (II) Preparation of flow cytometric samples

### (1) sample staining

Each of the flow tubes is added with 100µl cell suspension, and added with flow cytometric antibody for labeling lymphocyte subpopulations, gently shookin a vortex mixer at 2-8°C, and incubated without light for 10-30 minutes, preferably 15-20 minutes and most preferably 20 minutes.

### (2) sample washing and solution adding

After staining, the cells are resuspended in 2 ml pre-cooled PBS solution, and centrifuged at 1500-2900 rpm for 5-10 min. The supernatant is discarded, and 400µl PBS-EDTA solution at 2-8°C is added under stirring.

The present application solves the technical problems existing in the prior art. In the present application, blood plasma is separated by centrifugation, monocytes are purified by using lymphocyte separation solution, and the antibody is incubated and washed. At present, this method adopts lymphocyte separation solution and auxiliary centrifugation, and performs quantification according to the counting of lymphocytes, that is, adding a specified amount of lymphocytes and adding the antibody in an amount corresponding thereto, thereby solving the problem existing in the prior art, while avoiding a secondary binding caused by oversaturation of the antibody, or insufficient amount of antibody due to excessive lymphocytes.

Compared with traditional samples, the present application enriches and purifies lymphocyte subpopulations, so that part of background cell subpopulations such as granulocytes, red blood cells, platelets, etc. are removed, providing the detection with the advantages of obvious lymphocyte subpopulations, clean background, easy compensation, reduced amount of antibody, and more accurate and convenient flow cytometric analysis and detection. The present application saves time and the use of antibody, reduces the detection cost, makes the background clean, the clustering is clear and obvious, and the trace of the subsets such as DC cells are enriched, and the clustering is obvious, so that the detection results are accurate.

### Brief Description of Drawings

Fig. 1: flow cytometric diagram of a sample obtained with method of literature using hemolysin, i.e., lysis method, in example 1;
Fig. 2: flow cytometric diagram of a sample obtained with the present application , in example 1;
Fig. 3: flow cytometric diagram of a sample obtained with the present application, in example 1;
Fig. 4: flow cytometric diagram of a sample obtained with method of literature in example 1;
Fig. 5: flow cytometric diagram of a sample obtained with the present application, in example 1;
Fig. 6: a comparison diagram of the cell populations of the sample obtained according to the method of example 1 in Test example 2 and the sample obtained with lysis method of literature, (the upper 3 pictures are the flow cytometry diagrams obtained by the lysis method, and the lower 3 pictures are graphs correspond to the flow cytometry obtained by the sample separation method).;
Fig. 7: comparative diagram of cell enrichment in the sample obtained by the method of example 1 and the sample obtained by the lysis method of literature in test example 2;
Fig. 8: comparative diagram of enrichment of different types of cells in the sample obtained by the method of example 1 and the sample obtained with lysis method of literature in test example 2.

### Detailed description

The following examples and test examples are used to further elaborate the application, not intended to limit the effective scope of the present application in any way.

### Example 1 Preparation of PBMC samples needed for flow cytometry

### 1. Purpose

preparing PBMC samples needed for flow cytometric detection (also referred to as "samples" in the context of the present invention)

### 2. Principle

There are relatively complete antigens or receptors remained on the surface of living cells. Fluorescence labeled antibodies are used to bind to corresponding antigens on the cell surface, and fluorescence intensity and positive percentage are measured to provide the density and distribution of the corresponding antigens.

### 3. Preparation of instruments and consumable materials

### 3.1 Instruments

Super Clear Workbench, Era Beili low speed centrifuge DT5-4, a blood cell analyzer, a vortex mixer, and pipettes of various ranges

### 3.2 Consumable materials

15ml centrifuge tube, 10ml pipette, 1.5ml centrifuge tube, a flow tube, 3ml pasteurizer pipette, and pipette tips of various ranges.

### 3.3 Formulations of reagents

| Name | Stock solution | Formulation |
|---|---|---|
| 1×PBS | 10×PBS | stock solution: purified water =1:9 |
| 0.05-5% formalin | 10% formalin | stock solution: 1×PBS = 1:9 |
| PBS solution | 1×PBS | 1×PBS (0.01M PBS, pH=7.2~7.4) |
| PBS-EDTA solution | 1×PBS | adding 0.5M EDTA to 1×PBS (0.01M PBS; pH=7.2~7.4) until the final concentration of EDTA is 2.5mM |

Routine flow cytometric antibodies: anti-CD3 antibody, lin1[Lineage cocktail 1], anti-CD123 antibody and anti-CD11 antibody.

### 4. Operation procedure (illustrated by taking 5ml anticoagulant blood sample as an example)

### 4.1. separation of PBMC from peripheral blood

(1) obtaining 0.2ml original blood and counting;
(2) adding the rest of the original blood to a centrifuge tube, centrifuging at 1500-2900rpm for 15-20min, and removing upper plasma;
(3) diluting cell precipitate of the rest of the original blood with PBS solution by volume;
(4) providing a centrifuge tube and adding lymphocyte separation solution having a volume equal to the volume of the blood cell dilution to the centrifuge tube by using a pipette;
(5) adding blood cell dilution to the centrifuge tube in step (4) slowly for keeping the layer of separation solution clear;
(6) slowly placing the centrifuge tube into a centrifuge, balancing, and centrifuging at 1500-2900rpm for 15-20min;
(7) slowly suctioning and discarding the supernatant with a pipette after centrifuging;
(8) pipetting middle buffy coat cells into a new centrifuge tube, adding PBS solution to a volume of 10ml, counting 100µl of the solution with a whole blood cell counter for subsequent dilution, and centrifuging the rest of the cell suspension at 1500-2900rpm for 5-10min;
(9) discarding the supernatant after centrifuging, and removing residual liquid by using a 1ml pipette. According to the result of counting, appropriate amount of pre-cooled PBS solution at 4°C was added to adjust the density of cells to 1.5-3.0×10⁶/ml, for example, 2× 10⁶/ml.

### 4.2 Preparation of flow cytometric samples (the temperature was controlled in this operation, and the samples and antibodies were operated in an ice box throughout the operation)

### (1) Sample staining

A flow tube was added with 100µl lymphocyte suspension, added with flow cytometric antibody for labeling lymphocyte subpopulations, mixed evenly to homogenize the antibody and the cells, and incubated at 2-8°C without light for 20 minutes.

### (3) Sample washing

After staining, the cells were resuspended in 2ml pre-cooled PBS solution, and centrifuged at 1500-2900 rpm for 5-10 minutes.

### (4) Completion of samples

① If the detection on a flow cytometer is performed immediately, the supernatant is discarded and the cells are mixed in 400µl pre-cooled PBS-EDTA solution for flow detection;
② If the detection cannot be performed immediately, 500µl 0.05-5% formalin is added, fully mixed, and left to stand at 2-8°Cwithout light. Before detection, each of the tubes is added with 2ml PBS solution, and centrifuged at 1500-2900 rpm for 5-10 minutes to discard the supernatant, and the cells are evenly mixed with 400µl pre-cooled PBS-EDTA solution for detection on a flow cytometer.

### Test example 1 Comparative test between a sample prepared by the present application and a sample obtained by the method of treating with hemolysin

### 1.1 samples and reagents:

Lymphocyte sample of the present application: the sample was prepared from in vitro blood sample by using the method according to the above example 1;
Comparative lymphocyte samples: the sample was prepared from in vitro blood sample by hemolysin treatment of in vitro blood sample specified in section 1.2.1
5ml anticoagulant blood sample;
PBS solution: prepared by 10×PBS solution: purified water = 1:9 by volume
PBS-EDTA solution: prepared by adding 0.5M EDTA to 1 × PBS until the final concentration of EDTA is 2.5mM;
Routine flow cytometric antibodies: anti-CD3 antibody, lin1[Lineage Cocktail 1], anti-CD123 antibody, and anti-CD11 antibody.

### 1.2 test method:

### 1.2.1 hemolysin treatment method:

(1) sample staining: adding 100µl anticoagulant blood sample into each of the flow tubes, adding flow cytometric antibody into the tube to label lymphocyte subpopulations, slowly shaking the tube in a vortex mixer to mix the antibody and the cells evenly, and incubating the tube without light at 2-8°C for 20 minutes;
(2) lysing: adding 2 ml 1×BD hemolysin (i.e. erythrocyte lysis solution) to the flow tube for 5 minutes;
(3) sample washing: after 5 minutes, centrifuging the tube at 1500-2900 rpm for 10 minutes, discarding the supernatant after centrifuging, adding 2ml pre-cooled PBS solution to resuspend the cells, centrifuging the tube at 1500-2900 rpm for 5-20 minutes, discarding the supernatant, adding 400 µL PBS-EDTA solution at 2-8°C into the tube, and vortex mixing for 3 seconds to obtain the comparative lymphocyte sample.

### 1.2.2 detection method on flow cytometer:

a) checking the status of the cytometer before starting up;
b) warming up the flow cytometer system for 5-10 minutes and performing perfusion;
c) performing quality control procedure and proceeding to the next step after qualification;
d) setting the parameters of the flow cytometer, adjusting the voltage of each of detection channels, and setting the liquid flow speed to 35-40 µL/min;
e) vortex vibrating the prepared lymphocyte samples of the present application and the comparative lymphocyte samples for 3 seconds, and respectively subjecting the samples to detection under the conditions of: 15000 cells in the door; and collecting samples.

### 1.3 results of experiments:

The comparative observation results of the sample prepared by the present application and the sample treated by hemolysin are as follow:

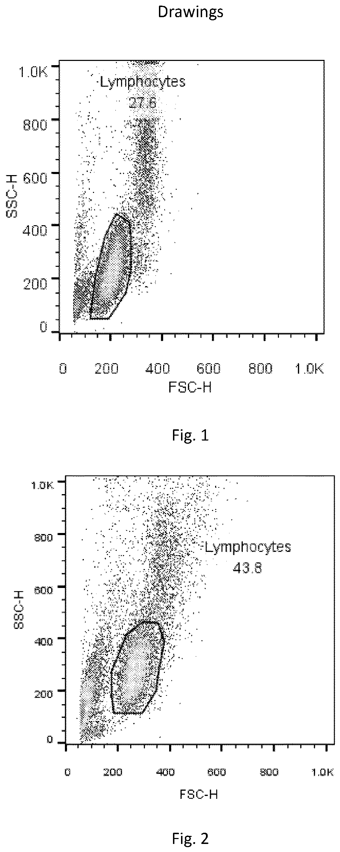

1.3.1 the sample treated with hemolysin provides the following observation results: it can be seen that, in the obtained cell populations, the number of cells is relatively small, the cell populations are too close to each other, and some of the granulocytes and lymphocytes adhere seriously, so that the cell populations cannot be identified; and there is a relatively large number of discrete cell debris, as particularly shown in Fig. 1.

The sample obtained by the present application provides the following observation results: after separation and enrichment, the number of cells is relatively large, the populations are obviously separated, the boundary is clear, and the part of the cell populations adhered to the granulocytes are removed during treatment, as particularly shown Fig. 2.

It can be seen by comparing the cells obtained in Fig. 1 and Fig. 2 that, the sample of the present application had successfully achieved separation and enrichment of cells, with a relatively large number of cells, obviously separated populations and clear boundaries, and the part of the cell populations adhered to the granulocytes were removed during treatment.

1.3.2 the comparative observation results of enriched cells from the sample prepared by the present application and the sample treated with hemolysin are as follow:

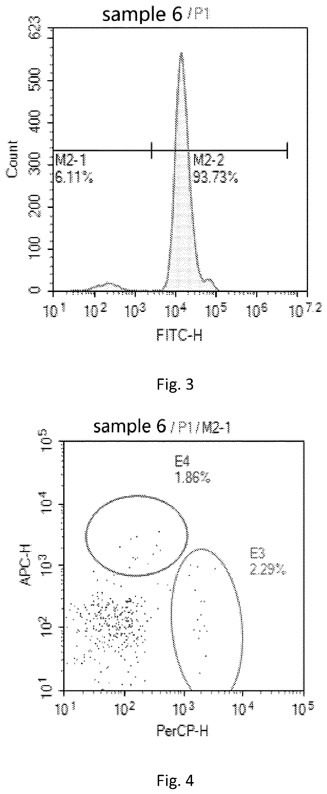

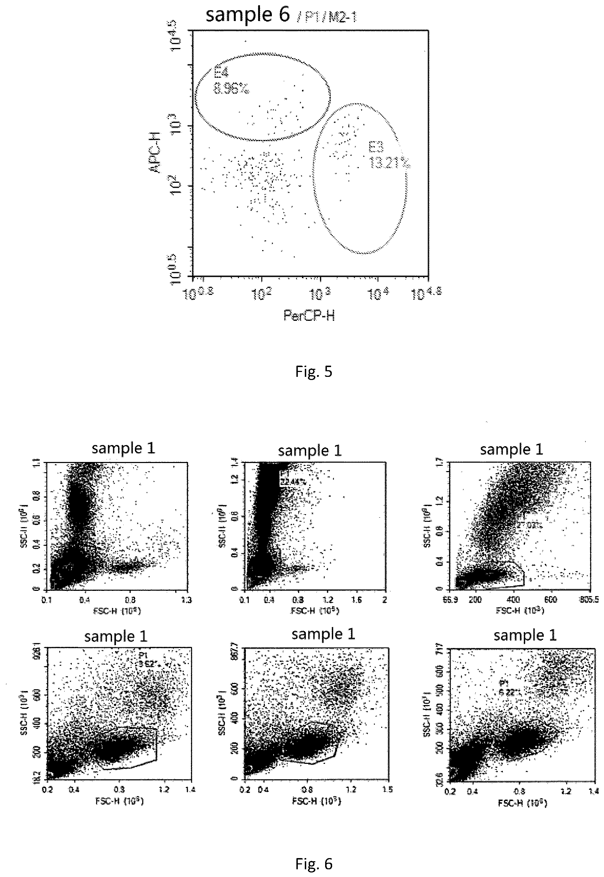

Figures 3, 4 and 5 show stained antibody lin1[Lineage Cocktail 1], anti-CD123 and anti-CD11; FITC is the first channel, and the stained antibody is antibody lin1[Lineage Cocktail 1]; PerCP is the third channel, and the stained antibody is anti-CD123; APC is the fourth channel, and the stained antibody is anti-CD11. In particular, Fig. 4 is the cell diagram obtained by hemolysin method; Fig. 5 is the flow cytometric diagram obtained by lymphocyte separation solution and centrifugation method. It is apparent that the cells in Fig. 5 are enriched in comparison with those in Fig. 4.

In terms the number of cells, the sample of the present application has a better effect of cell enrichment. The particular analysis process is as follows: first, drawing a histogram, circling door 1: FL1 -, then circling FL3-H, FL4-H cell subpopulation-1 inside door 1, that is, cell surface marker lin1⁻CD123⁺CD11C⁻ represents plasma cell-derived DC cells (pDC);

the cell surface marker lin1⁻CD11C⁺ represents myeloid cell-derived DC cells (mDC); in particular, in the field of flow cytometry detection, the more cells can be collected, the more accurate the results will be. It is obvious that the number and populations of mDC cells and pDC cells in Fig. 5 are better than those in Fig. 4.

### Test example 2 Comparison between a sample prepared by the present application and a sample obtained by hemolysin lysis

### 2.1 samples and reagents:

Lymphocyte sample of the present application: the sample was prepared from in vitro blood sample by using the method according to the above example 1;
Comparative lymphocyte samples: the sample was prepared from in vitro blood sample by a method specified in the following section 2.2.1
5ml anticoagulant blood sample;
PBS solution: prepared by 10×PBS solution: purified water = 1:9 by volume
PBS-EDTA solution: prepared by adding 0.5M EDTA to 1×PBS until the final concentration of EDTA is 2.5mM;
Routine flow cytometric antibodies: anti-CD3 antibody, anti-CD123 antibody, anti-CD11 antibody, CD3 FITC, CD8PerCP, and CD4PerCP.

### 2.2 test method:

### 2.2.1 hemolysin lysis treatment method:

(1) sample staining: adding 100µl anticoagulant blood sample into each of the flow tubes, adding flow cytometric antibody to the tube to label lymphocyte subpopulations, fully mixing the antibody and the cells, and incubating the tube at 2-8°C without light for 20 minutes;
(2) lysing: adding 2ml 1×BD hemolysin (i.e. erythrocyte lysis solution) to the flow tube for 5 minutes;
(3) sample washing: after 5 minutes, centrifuging the tube at 1500rpm for 10 minutes, discarding the supernatant after centrifuging, adding 2ml pre-cooled PBS solution to resuspend the cells, centrifuging the tube at 1500 rpm for 5 minutes, discarding the supernatant, adding 400 µL PBS-EDTA solution at 2-8°C into the tube, and mixing evenly.

### 2.2.2 detection method on flow cytometer:

a) checking the status of the cytometer before starting up;
b) warming up the flow cytometer system for 5-10 minutes and performing perfusion;
c) performing quality control procedure and proceeding to the next step after qualification;
d) setting the parameters of the flow cytometer, adjusting the voltage of each of detection channels, and setting the liquid flow speed to 35-40 µL/min;
e) vortex vibrating the prepared lymphocyte samples of the present application and the comparative lymphocyte samples for 3 seconds, and respectively subjecting the samples to detection under the conditions of: 15000 cells in the door; and collecting samples.

### 2.3 results of experiments:

2.3.1 From the comparison, it can be seen that, the samples obtained by lysis had too much debris and unclean background, which will influence the collection and analysis of cytometer data, and might cause bias to the compensation adjustment of lymphocyte (see Fig. 6).

In particular, from the observation of lymphocyte cells inside the door of the circle in the analysis figure of lymphocyte subpopulations obtained by lysis, it can be seen that the lymphocyte populations and cell debris populations are separated far from ideal (see three figures in the first row of Fig. 6).

The population inside the door of the circle in the analysis figure of lymphocyte subpopulations obtained by the present application is lymphocyte population, and it can be seen that the lymphocyte populations and cell debris populations are widely separated, being very ideal (see three figures in the second row of Fig. 6).

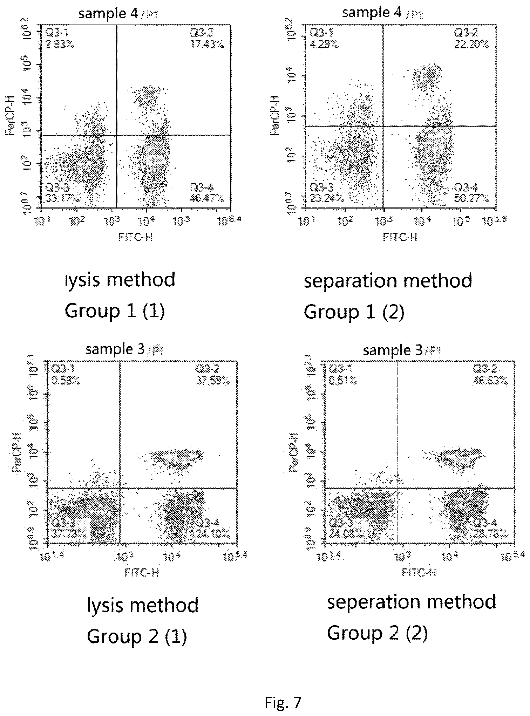

2.3.2 It can be seen from Fig. 7 that, when using fluorescence antibody, the present application saves 70% or more of the antibodies by adding fluorescent antibody in proportion based on the counted number of lymphocytes. The use amount of the combined antibody during detection in the present application is 15µl, and the use amount of the antibody in the lysis method is 60µl. The method of the present application does not cause excessive or insufficient amount of antibody. From Fig. 7, it can be seen that what are labeled in Group 1 are CD3 FITC and CD8PerCP; and what are labeled in Group 2 are CD3 FITC and CD4PerCP. Due to the different samples obtained by different treatment methods, for the same in vitro peripheral blood sample, the results are that the proportion of double positive groups in the samples obtained by the lysis method is obviously reduced, and the amount of antibody cannot be quantified. The samples obtained by the method of the present application can be added with antibody by an amount corresponding to that of the cells. Besides, it is obvious that the cells obtained by separation method have been enriched to a certain extent.

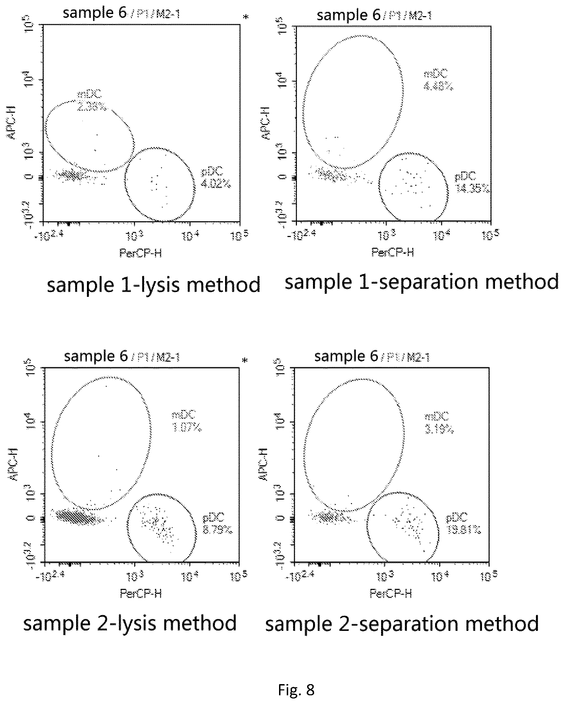

2.3.3 it can be seen from Fig. 8 that, the sample prepared by the present application has enrichment effect for the sample with small proportion of cell subpopulations. The proportion of cell subpopulation MDC and PDC in the same in vitro peripheral blood samples is relatively small. The observation results of the samples obtained by lysis method and the present method are different, and the cell subpopulations in the samples obtained by the present application are enriched, which can be shown from the number and percentage of cells.

## Claims

1. A method for preparing flow cytometric sample in human immune cells in vitro for flow cytometry, **characterized by** comprising the steps of:
1) separating plasma and blood precipitation by centrifuging in vitro anticoagulant blood samples to obtain blood cell precipitate;
2) mixing the blood cell precipitate with PBS solution to obtain a blood cell dilution;
3) adding the blood cell dilution into a centrifuge tube containing the lymphocyte separation solution having a volume equal to that of the blood cell dilution, centrifuging, and discarding plasma to obtain buffy coat cells;
4) adding the buffy coat cells to a centrifuge tube, adding PBS solution under stirring, centrifuging, discarding supernatant, and then adding PBS solution to adjust the cell concentration to provide lymphocyte suspension having a concentration of 1×10⁵/ml - 1×10⁸/ml, further preferably 1.5×10⁶/ml - 3×10⁶/ml;
5) adding the lymphocyte suspension into a flow tube, then adding flow cytometric antibody to label lymphocyte subpopulations, mixing evenly, and incubating at 2-8°C without light;
6) adding PBS solution to the flow tube, mixing, and centrifuging to remove unbound antibody;
7) completion of samples;
① if a detection on a flow cytometer is performed immediately, the supernatant is discarded and the precipitate is mixed with PBS-EDTA solution at 2-8°C for flow cytometric detection; or
② if the detection cannot be performed immediately, formalin is added, mixed, and left to stand at 2-8°Cwithout light; and before detection, each of the tubes is added with PBS solution, centrifuged to discard the supernatant, and evenly mixed with PBS-EDTA solution at 2-8°C for flow cytometric detection.

2. The method according to claim 1, **characterized in that**, the PBS solution is 0.005-0.05M PBS solution at pH 7.2-7.4; and the PBS-EDTA solution is a mixed solution at pH 7.2-7.4 containing 0.005-0.05M PBS and a final concentration of 2-3mM EDTA.

3. The method according to claim 1, **characterized in that**, step 1 further comprises centrifuging conditions of 1500-3500rpm, 5-30min; preferably 1500-2900rpm and 15-20min.

4. The method according to claim 1, **characterized in that**, in step 2), the blood cell precipitate and PBS solution are added in a volume ratio of 1:0.5-1:2 and preferably 1:1.

5. The method according to claim 1, **characterized in that**, step 3) further comprises centrifuging conditions of 1500-3500rpm, 10-20min, 4°C; preferably 1500-2900rpm, 20min, 4°C.

6. The method according to claim 1, **characterized in that**, step 4) further comprises centrifuging conditions of 1500-3500rpm for 5-20 minutes; preferably 1500-2900rpm for 5-10 minutes.

7. The method according to claim 1, **characterized in that**, step 6) further comprises centrifuging conditions of 1500-2900rpm for 5-10 minutes.

8. The method according to claim 1, **characterized in that**, the amount of PBS solution added in step 6) is an amount equal to the volume of blood cell precipitate.

9. The method according to claim 1, **characterized in that**, the completion of samples in step 7) further comprises:
① if a detection on a flow cytometer is performed immediately, the supernatant is discarded and the precipitate is mixed with PBS-EDTA solution at 2-8°C for flow cytometric detection; or
② if the detection is not be performed immediately, 0.05-5% formalin, preferably 1% formalin, is added, mixed, and left to stand at 2-8°Cwithout light; and before detection, each of the tubes is added with PBS solution, centrifuged to discard the supernatant, and evenly mixed with PBS-EDTA solution at 2-8°C for flow cytometric detection.

10. The method according to claim 9, **characterized in that**, the PBS solution is 0.005-0.05M PBS solution at pH 7.2-7.4; and the PBS-EDTA solution is a mixed solution at pH 7.2-7.4 containing 0.005-0.05M PBS and a final concentration of 2-3mM EDTA.

11. The method according to any one of claims 1-10, **characterized in that**, the steps 1) to 6) further comprise:
(I) separation of human peripheral blood mononuclear cells
(1) obtaining an in vitro anticoagulant peripheral blood sample and counting;
(2) centrifuging the blood sample in a centrifuge tube and discarding the upper plasma to provide lower blood cell precipitate;
(3) diluting the blood cell precipitate with PBS solution by a volume ratio of 1:1 to provide blood cell dilution;
(4) adding lymphocyte separation solution having a volume equal to the volume of the blood cell dilution into a centrifuge tube;
(5) adding the blood cell dilution into the centrifuge tube in step (4) slowly for keeping the layer of the separation solution clear;
(6) Slowly placing the centrifuge tube into a low-speed centrifuge, balancing, and centrifuging;
(7) discarding the supernatant after centrifuging to provide buffy coat cells;
(8) placing the buffy coat cells into a centrifuge tube, adding PBS solution, counting, and centrifuging; and
(9) removing the supernatant after centrifuging, and adding PBS solution at 2-8°C according to the result of counting, to obtain a cell suspension having a cell density of 1.5-3.0×10⁶/ml;
(II) preparation of flow cytometric samples
(10) sample staining
added 100µl lymphocyte suspension into a flow tube, adding corresponding antibody, mixed evenly, and incubating at 2-8°C without light for 10-30 minutes, preferably 15-20 minutes, and most preferably 20 minutes;
(11) sample washing
after dyeing, adding PBS solution pre-cooled at 2-8°C to resuspend the cells, centrifuging for 5-10 minutes, and removing unbound antibody.

## Patentansprüche

1. Verfahren zur Herstellung einer durchflusszytometrischen Probe bei menschlichen Immunzellen in vitro für die Durchflusszytometrie, **dadurch gekennzeichnet, dass** folgende Schritte durchgeführt werden:
1) Trennen von Plasma und Blutfällungsprodukt, indem in vitro gerinnungsgehemmte Blutproben zentrifugiert werden, um einen Blutzellenniederschlag zu erhalten;
2) Mischen des Blutzellenniederschlags mit PBS-Lösung, um verdünnte Blutzellen zu erhalten;
3) Zugeben der verdünnten Blutzellen in ein Zentrifugenröhrchen, welches die Lymphozyten-Trennlösung enthält, deren Volumen demjenigen der verdünnten Blutzellen entspricht, Zentrifugieren und Verwerfen des Plasmas, um Buffy-Coat-Zellen zu erhalten;
4) Zugeben der Buffy-Coat-Zellen in ein Zentrifugenröhrchen, Zugeben von PBS-Lösung unter Rühren, Zentrifugieren, Verwerfen des Überstands und anschließendes Zugeben von PBS-Lösung zur Anpassung der Zellkonzentration, um eine Lymphozytensuspension mit einer Konzentration von 1 × 10⁵/mL bis 1 × 10⁸/mL, stärker bevorzugt 1,5 × 10⁶/mL bis 3 × 10⁶/mL, zu erhalten;
5) Zugeben der Lymphozytensuspension in ein Durchflussröhrchen und anschließendes Hinzufügen von durchflusszytometrischen Antikörpern, um Lymphozyten-Subpopulationen zu markieren, gleichmäßiges Mischen und Inkubieren bei 2 bis 8 °C unter Lichtausschluss;
6) Zugeben von PBS-Lösung in das Durchflussröhrchen, Vermischen und Zentrifugieren, um ungebundene Antikörper zu entfernen;
7) Abschließen der Probenaufbereitung;
1. wenn unverzüglich ein Nachweis auf einem Durchflusszytometer erfolgt, wird der Überstand verworfen und der Niederschlag mit einer PBS-EDTA-Lösung bei 2 bis 8°C für den durchflusszytometrischen Nachweis gemischt; oder
2. wenn der Nachweis nicht unverzüglich durchgeführt werden kann, wird Formaldehyd zugegeben, vermischt und unter Lichtausschluss bei 2 bis 8°C stehen gelassen; wobei weiterhin vor dem Nachweis PBS-Lösung in jedes der Röhrchen gegeben und zentrifugiert wird, woraufhin der Überstand verworfen wird und eine gleichmäßige Durchmischung mit PBS-EDTA-Lösung bei 2 bis 8°C für den durchflusszytometrischen Nachweis erfolgt.

2. Verfahren gemäß Anspruch 1, **dadurch gekennzeichnet, dass** es sich bei der PBS-Lösung um eine Lösung von 0,005 bis 0,05 M an PBS bei pH 7,2 bis 7,4 handelt; und es sich bei der PBS-EDTA-Lösung um eine Mischlösung bei pH 7,2 bis 7,4 handelt, die 0,005 bis 0,05 M an PBS und eine Endkonzentration von 2 bis 3 mM an EDTA enthält.

3. Verfahren gemäß Anspruch 1, **dadurch gekennzeichnet, dass** Schritt 1 weiterhin Zentrifugierbedingungen von 1500 bis 3500 U/min., 5 bis 30 min.; vorzugsweise von 1500 bis 2900 U/min. und 15 bis 20 min. umfasst.

4. Verfahren gemäß Anspruch 1, **dadurch gekennzeichnet, dass** in Schritt 2) der Blutzellenniederschlag und die PBS-Lösung in einem Volumenverhältnis von 1:0,5 bis 1:2 und vorzugsweise von 1:1 zugegeben werden.

5. Verfahren gemäß Anspruch 1, **dadurch gekennzeichnet, dass** Schritt 3) weiterhin Zentrifugierbedingungen von 1500 bis 3500 U/min., 10 bis 20 min., 4°C; vorzugsweise von 1500 bis 2900 U/min., 20 min., 4°C umfasst.

6. Verfahren gemäß Anspruch 1, **dadurch gekennzeichnet, dass** Schritt 4) weiterhin Zentrifugierbedingungen von 1500 bis 3500 U/min. für 5 bis 20 Minuten; vorzugsweise von 1500 bis 2900 U/min. für 5 bis 10 Minuten umfasst.

7. Verfahren gemäß Anspruch 1, **dadurch gekennzeichnet, dass** Schritt 6) weiterhin Zentrifugierbedingungen von 1500 bis 2900 U/min für 5 bis 10 Minuten umfasst.

8. Verfahren gemäß Anspruch 1, **dadurch gekennzeichnet, dass** die in Schritt 6) zugegebene Menge an PBS-Lösung eine Menge ist, die gleich dem Volumen des Blutzellenniederschlags ist.

9. Verfahren gemäß Anspruch 1, **dadurch gekennzeichnet, dass** das Abschließen der Probenaufbereitung in Schritt 7) weiterhin Folgendes umfasst:
1. wenn unverzüglich ein Nachweis auf einem Durchflusszytometer erfolgt, wird der Überstand verworfen und der Niederschlag mit einer PBS-EDTA-Lösung bei 2 bis 8°C für den durchflusszytometrischen Nachweis gemischt; oder
2. wenn der Nachweis nicht unverzüglich durchgeführt wird, erfolgt eine Zugabe von 0,05 bis 5% Formaldehyd, vorzugsweise 1% Formaldehyd, woraufhin vermischt und unter Lichtausschluss bei 2 bis 8°C stehen gelassen wird; wobei weiterhin vor dem Nachweis PBS-Lösung in jedes der Röhrchen gegeben und zentrifugiert wird, um den Überstand zu verwerfen, und eine gleichmäßige Durchmischung mit PBS-EDTA-Lösung bei 2 bis 8°C für den durchflusszytometrischen Nachweis erfolgt.

10. Verfahren gemäß Anspruch 9, **dadurch gekennzeichnet, dass** es sich bei der PBS-Lösung um eine Lösung von 0,005 bis 0,05 M an PBS bei pH 7,2 bis 7,4 handelt; und es sich bei der PBS-EDTA-Lösung um eine Mischlösung bei pH 7,2 bis 7,4 handelt, die 0,005 bis 0,05 M an PBS und eine Endkonzentration von 2 bis 3 mM an EDTA enthält.

11. Verfahren gemäß einem der Ansprüche 1 bis 10, **dadurch gekennzeichnet, dass** die Schritte 1) bis 6) weiterhin Folgendes umfassen:
I) Abtrennen von mononukleären Zellen des menschlichen peripheren Blutes
(1) Gewinnen in vitro einer gerinnungsgehemmten Probe peripheren Blutes und Auszählen;
(2) Zentrifugieren der Blutprobe wird in einem Zentrifugenröhrchen und Verwerfen des oben befindlichen Plasmas, um die unten befindlichen Blutzellen als Niederschlag zu erhalten;
(3) Verdünnen des Blutzellenniederschlags mit PBS-Lösung im Volumenverhältnis 1:1, um verdünnte Blutzellen zu erhalten;
(4) Zugeben einer Lymphozyten-Trennlösung, deren Volumen dem Volumen der verdünnten Blutzellen entspricht, in ein Zentrifugenröhrchen;
(5) langsames Zugeben der verdünnten Blutzellen in das Zentrifugenröhrchen des Schrittes (4), damit die Schicht der Trennlösung klar bleibt;
(6) Langsames Einlegen des Zentrifugenröhrchens in eine Zentrifuge mit geringer Geschwindigkeit, Ausbalancieren und Zentrifugieren;
(7) Verwerfen des Überstands nach dem Zentrifugieren, um Buffy-Coat-Zellen zu erhalten;
(8) Überführen der Buffy-Coat-Zellen in ein Zentrifugenröhrchen, Zugeben von PBS-Lösung, Auszählen und Zentrifugieren; und
(9) Entfernen des Überstands nach dem Zentrifugieren und Zugeben von PBS-Lösung bei 2 bis 8°C in Abhängigkeit von dem Auszählwert, um eine Zellsuspension mit einer Zelldichte von 1,5 bis 3,0 × 10⁶/mL zu erhalten;
II) Aufbereiten durchflusszytometrischer Proben
(10) Anfärben der Proben
Zugeben von 100 µL Lymphozytensuspension in ein Durchflussröhrchen, Zugeben des entsprechenden Antikörpers, gleichmäßiges Durchmischen, und Inkubieren bei 2 bis 8 °C unter Lichtausschluss für 10 bis 30 Minuten, vorzugsweise 15 bis 20 Minuten, und mit dem größten Vorzug 20 Minuten;
(11) Waschen der Proben
nach dem Anfärben, Zugeben von PBS-Lösung, die bei 2 bis 8 °C vorgekühlt wurde, um die Zellen wieder in Suspension zu bringen, 5 bis 10 Minuten lang Zentrifugieren und Entfernen ungebundener Antikörper.

## Revendications

1. Procédé pour la préparation d'un échantillon cytométrique en flux dans des cellules immunitaires humaines *in vitro* pour une cytométrie en flux, **caractérisé par le fait qu'**il comprend les étapes de :
1) séparation d'une précipitation de plasma et de sang par centrifugation *in vitro* d'échantillons de sang à anticoagulant pour obtenir un précipité de cellules sanguines ;
2) mélange du précipité de cellules sanguines avec une solution de PBS pour obtenir une dilution de cellules sanguines ;
3) ajout de la dilution de cellules sanguines dans un tube de centrifugeuse contenant une solution de séparation de lymphocytes ayant un volume égal à celui de la dilution de cellules sanguines, centrifugation, et rejet de plasma pour obtenir des cellules de la couche leucocytaire ;
4) ajout des cellules de la couche leucocytaire à un tube de centrifugeuse, ajout d'une solution de PBS sous agitation, centrifugation, rejet de surnageant, puis ajout d'une solution de PBS pour ajuster la concentration en cellules pour fournir une suspension de lymphocytes ayant une concentration de 1 × 10⁵/ml à 1 × 10⁸/ml, en outre préférablement 1,5 × 10⁶/ml à 3 × 10⁶/ml ;
5) ajout de la suspension de lymphocytes dans un tube de flux, puis ajout d'un anticorps cytométrique en flux pour marquer des sous-populations de lymphocytes, mélange de manière uniforme, et incubation à une température de 2 à 8 °C sans lumière ;
6) ajout d'une solution de PBS au tube de flux, et centrifugation pour éliminer un anticorps non lié ;
7) finalisation des échantillons ;
① si une détection sur un cytomètre en flux est réalisée immédiatement, le surnageant est rejeté et le précipité est mélangé avec une solution de PBS-EDTA à une température de 2 à 8 °C pour une détection cytométrique en flux ; ou
② si la détection ne peut pas être réalisée immédiatement, de la formaline est ajoutée, mélangée et laissée à reposer à une température de 2 à 8 °C sans lumière ; et avant détection, une solution de PBS est ajoutée à chacun des tubes, qui est centrifugé pour rejeter le surnageant et mélangé de manière uniforme avec une solution de PBS-EDTA à une température de 2 à 8 °C pour une détection cytométrique en flux.

2. Procédé selon la revendication 1, **caractérisé en ce que** la solution de PBS est une solution de PBS 0,005 à 0,05 M à pH 7,2 à 7,4 ; et la solution de PBS-EDTA est une solution mélangée à pH 7,2 à 7,4 contenant 0,005 à 0,05 M de PBS et une concentration finale de 2 à 3 mM d'EDTA.

3. Procédé selon la revendication 1, **caractérisé en ce que** l'étape 1 comprend en outre des conditions de centrifugation de 1 500 à 3 500 tpm, 5 à 30 min ; préférablement 1 500 à 2 900 tpm et 15 à 20 min.

4. Procédé selon la revendication 1, **caractérisé en ce que** dans l'étape 2), le précipité de cellules sanguines et la solution de PBS sont ajoutés en un rapport en volume de 1 : 0,5 à 1 : 2 et préférablement 1 : 1.

5. Procédé selon la revendication 1, **caractérisé en ce que** l'étape 3) comprend en outre des conditions de centrifugation de 1 500 à 3 500 tpm, 10 à 20 min, 4 °C ; préférablement 1 500 à 2 900 tpm, 20 min, 4 °C.

6. Procédé selon la revendication 1, **caractérisé en ce que** l'étape 4) comprend en outre des conditions de centrifugation de 1 500 à 3 500 tpm pendant 5 à 20 minutes ; préférablement 1 500 à 2 900 tpm pendant 5 à 10 minutes.

7. Procédé selon la revendication 1, **caractérisé en ce que** l'étape 6) comprend en outre des conditions de centrifugation de 1 500 à 2 900 tpm pendant 5 à 10 minutes.

8. Procédé selon la revendication 1, **caractérisé en ce que** la quantité de solution de PBS ajoutée dans l'étape 6) est une quantité égale au volume de précipité de cellules sanguines.

9. Procédé selon la revendication 1, **caractérisé en ce que** la finalisation des échantillons dans l'étape 7) comprend en outre :
① si une détection sur un cytomètre en flux est réalisée immédiatement, le surnageant est rejeté et le précipité est mélangé avec une solution de PBS-EDTA à une température de 2 à 8 °C pour une détection cytométrique en flux ; ou
② si la détection ne peut pas être réalisée immédiatement, 0,05 à 5 % de formaline, préférablement 1 % de formaline, est ajoutée, mélangée et laissée à reposer à une température de 2 à 8 °C sans lumière ; et avant détection, une solution de PBS est ajoutée à chacun des tubes, qui est centrifugé pour rejeter le surnageant et mélangé de manière uniforme avec une solution de PBS-EDTA à une température de 2 à 8 °C pour une détection cytométrique en flux.

10. Procédé selon la revendication 9, **caractérisé en ce que** la solution de PBS est une solution de PBS 0,005 à 0,05 M à pH 7,2 à 7,4 ; et la solution de PBS-EDTA est une solution mélangée à pH 7,2 à 7,4 contenant 0,005 à 0,05 M de PBS et une concentration finale de 2 à 3 mM d'EDTA.

11. Procédé selon l'une quelconque des revendications 1 à 10, **caractérisé en ce que** les étapes 1) à 6) comprennent en outre :
(I) la séparation de cellules mononucléaires de sang périphérique humain
(1) l'obtention d'un échantillon de sang périphérique à anticoagulant *in vitro* et un décompte ;
(2) la centrifugation de l'échantillon de sang dans un tube de centrifugeuse et le rejet du plasma supérieur pour fournir un précipité de cellules sanguines inférieur ;
(3) la dilution du précipité de cellules sanguines avec une solution de PBS par un rapport en volume de 1 : 1 pour fournir une dilution de cellules sanguines ;
(4) l'ajout d'une solution de séparation de lymphocytes ayant un volume égal au volume de la dilution de cellules sanguines dans un tube de centrifugeuse ;
(5) l'ajout de la dilution de cellules sanguines dans le tube de centrifugeuse dans l'étape (4) lentement pour maintenir limpide la couche de la solution de séparation ;
(6) le placement lentement du tube de centrifugeuse dans une centrifugeuse à faible vitesse, l'équilibration et la centrifugation ;
(7) le rejet du surnageant après la centrifugation pour fournir des cellules de la couche leucocytaire ;
(8) le placement des cellules de la couche leucocytaire dans un tube de centrifugeuse, l'ajout d'une solution de PBS, le décompte et la centrifugation ; et
(9) le rejet du surnageant après centrifugation, et l'ajout d'une solution de PBS à une température de 2 à 8 °C selon le résultat du décompte, pour obtenir une suspension de cellules ayant une densité de cellules de 1,5 à 3,0 × 10⁶/ml ;
(II) la préparation d'échantillons cytométriques en flux
(10) la teinture d'échantillons 100 µl d'une suspension de lymphocytes sont ajoutés dans un tube de flux, ajout d'un anticorps correspondant, mélangé de manière uniforme, et incubation à une température de 2 à 8 °C sans lumière pendant 10 à 30 minutes, préférablement 15 à 20 minutes, et le plus préférablement 20 minutes ;
(11) le lavage d'échantillons après coloration, ajout d'une solution de PBS pré-refroidie à une température de 2 à 8 °C pour mettre à nouveau en suspension les cellules, centrifugation pendant 5 à 10 minutes, et élimination d'anticorps non lié.
